# EUROPÄISCHE PATENTANMELDUNG

(11) **EP 0 709 357 A1**
(43) Veröffentlichungstag der Anmeldung: **01.05.1996**
(21) Anmeldenummer: 95115897.1
(22) Anmeldetag: 09.10.1995
(51) Int. Cl.: C07C 29/32, C07C 33/48, C07C 33/28, C07C 33/30, C07D 239/26

(54) **Verfahren zur Kreuzkupplung von aromatischen Halogenverbindungen oder Perfluoralkylsulfonaten mit terminalen Alkinen**

(30) Priorität: 28.10.1994 DE 4438586; 31.10.1994 DE 4438877
(71) Anmelder: HOECHST AKTIENGESELLSCHAFT, D-65929 Frankfurt am Main (DE)
(72) Erfinder: Haber, Steffen, Dr., D-76726 Germersheim (DE); Manero, Javier, Dr., D-65931 Frankfurt (DE); Beck, Gerhard, Dr., D-60320 Frankfurt (DE); Lagouardat, Jacques, Dr., F-93160 Noisy le Grand (FR)

(57) **Zusammenfassung**

Ein Verfahren zur Herstellung mehrkerniger aromatischer Verbindungen durch Kreuzkupplung von terminalen Alkinen mit aromatischen Halogenverbindungen oder - Perfluoralkylsulfonaten unter Palladiumkatalyse in Gegenwart mindestens eines wasserlöslichen Komplexliganden, dadurch gekennzeichnet, daß das Reaktionsmedium eine wäßrige und eine organische Phase bildet und das Palladium in Form einer in der organischen Phase löslichen Palladiumverbindung oder in fester Form als Palladiummetall, insbesondere auf einem Träger aufgebracht, zugesetzt wird.

Die erfindungsgemäße Reaktion verläuft chemoselektiv, so daß selbst elektrophile Gruppen, wie Ester oder Nitrile, den Verlauf der Reaktion nicht beeinträchtigen.

Durch das erfindungsgemäße Verfahren lassen sich organische Verbindungen ökonomisch in sehr guten Ausbeuten und gleichzeitig sehr hoher Reinheit, insbesondere ohne Verunreinigung durch die Komplexliganden, herstellen.

## Beschreibung

Die Erfindung betrifft ein Verfahren zur Kupplung von aromatischen Halogenverbindungen oder Perfluoralkylsulfonaten mit terminalen Alkinen unter Palladiumkatalyse in Anwesenheit von wasserlöslichen Komplexliganden.

Die palladiumkatalysierte Kreuzkupplungsreaktion von aromatischen Halogenverbindungen oder - Perfluoralkylsulfonaten mit terminalen Alkinen (siehe z.B. R. F. Heck, Palladium Reagents in Organic Syntheses, Academic Press, S. 260 ff.; J. P. Genet, E. Blart, M. Savignac, Synlett 1992, 715 oder G. T. Crisp, T. A. Robertson, Tetrahedron 1992, 48, 3239) wird seit einigen Jahren in steigendem Umfang in vielen Bereichen der organischen Synthese genutzt.

Bei den zitierten Verfahren handelt es sich um homogen katalysierte Prozesse unter Verwendung von Pd(II)-Komplexen, insbesondere Bis-(triphenylphosphan)-palladium(II)chlorid. Die Anwesenheit von phosphorhaltigen Komplexliganden steigert im allgemeinen Ausbeuten und Selektivität der Reaktion erheblich.

Ein Nachteil dieser Verfahren liegt jedoch bei den hohen Katalysatorkosten, die eine wirtschaftliche Überführung der Prozesse in einen größeren Produktionsmaßstab (kg, t) schwierig machen. Zudem wird eine Kontamination des Produktes und des Abfalls mit Phosphorverbindungen beobachtet, was insbesondere bei Wirkstoffen ein Nachteil sein kann.

Es ist auch bekannt, wasserlösliche Palladiumkomplexe für die obengenannten Kupplungsreaktionen einzusetzen und in rein wäßriger - oder Zweiphasensystemen aus organischer - und Wasserphase zu arbeiten (siehe z.B. US 5,043,510 oder J. P. Genet et al., Synlett 1992, 715). Dabei werden wasserlösliche Phosphanliganden, wie Triphenylphosphano-3,3',3''-trisulfonattrinatriumsalz (tppts), verwendet, um den wasserlöslichen Palladiumkomplex zu erhalten. Es ist jedoch beschrieben (A. L. Casalnuovo und J. C. Calabrese, J. Am. Chem. Soc. 1990, 112, 4324), daß die Ausbeuten im Zweiphasensystem deutlich niedriger als im Einphasensystem liegen.

Zudem berichten N. A. Bumagin, V. V. Bykor und I. P. Belets-Kaya, Izv. Akad. Nauk SSSR, Ser. Khim 1990, 2665, über niedrige Ausbeuten bei Verwendung von Palladium(II)acetat als Katalysator.

Weiterhin leiden die Prozesse unter den oben beschriebenen Nachteilen wie Katalysatorkosten und Verunreinigung des Produkts mit den Komplexliganden.

Aufgabe der vorliegenden Erfindung war es daher, ein ökonomisch günstiges Verfahren zur Kupplung von terminalen Alkinen mit aromatischen Perfluoralkylsulfonaten oder aromatischen Halogenverbindungen bereitzustellen, das die Kupplungsprodukte in sehr hoher Reinheit und guter Ausbeute liefert.

Es wurde nun überraschend gefunden, daß bei Umsetzung von terminalen Alkinen mit aromatischen Halogenverbindungen oder - Perfluoralkylsulfonaten in einem zweiphasigen Reaktionsmedium in Gegenwart einer Base, katalytischer Mengen einer in organischen Lösemitteln löslichen Palladiumverbindung und mindestens eines wasserlöslichen Komplexliganden Arylalkine in hervorragenden Ausbeuten und sehr hohen Reinheiten erhalten werden.

Gegenstand der Erfindung ist somit ein Verfahren zur Herstellung von 1-Arylalkinen durch Kreuzkupplung von terminalen Alkinen mit aromatischen Halogenverbindungen oder - Perfluoralkylsulfonaten unter Palladiumkatalyse in Gegenwart mindestens eines wasserlöslichen Komplexliganden, dadurch gekennzeichnet, daß das Reaktionsmedium eine wäßrige und eine organische Phase bildet und das Palladium in Form einer in der organischen Phase löslichen Palladiumverbindung oder in fester Form als Palladiummetall, insbesondere auf einem Träger aufgebracht, zugesetzt wird.

Die erfindungsgemäße Reaktion verläuft chemoselektiv, so daß selbst elektrophile Gruppen, wie Ester oder Nitrile, den Verlauf der Reaktion nicht beeinträchtigen.

Durch das erfindungsgemäße Verfahren lassen sich 1-Arylalkine ökonomisch in sehr guten Ausbeuten und gleichzeitig sehr hoher Reinheit, insbesondere ohne Verunreinigung durch die Komplexliganden, herstellen.

Die Löslichkeit der Palladiumverbindung in der organischen Phase beträgt vorzugsweise mindestens 0,5 mmol/l, besonders bevorzugt mindestens 5 mmol/l, insbesondere bevorzugt mindestens 20 mmol/l.

Das erfindungsgemäße Verfahren wird in einem Mehrphasensystem durchgeführt, das mindestens eine wäßrige Phase und eine organische Phase enthält. Die wäßrige Phase kann dabei neben Wasser auch ein oder mehrere wasserlösliche organische Lösungsmittel enthalten. Es können auch feste Phasen anwesend sein.

Zur Durchführung des erfindungsgemäßen Verfahrens werden die aromatische Halogenverbindung oder das Perfluoralkylsulfonat, das terminale Alkin, die Base, die katalytische Menge des Palladium oder der in organischen Lösungsmitteln löslichen Palladiumverbindung und ein wasserlöslicher Komplexligand in eine Mischung aus Wasser und einem oder mehreren an der Reaktion unbeteiligten organischen Lösungsmitteln gegeben und bei einer Temperatur von -10°C bis 200°C, bevorzugt bei 0°C bis 170°C, besonders bevorzugt bei 5°C bis 150°C, insbesondere bevorzugt bei 10 bis 120°C, für einen Zeitraum von 1 Stunde bis 100 Stunden, bevorzugt 5 Stunden bis 70 Stunden, besonders bevorzugt 5 Stunden bis 50 Stunden, gerührt.

Die Aufarbeitung erfolgt dann nach bekannten, dem Fachmann geläufigen Methoden.

Um eine Verunreinigung des Produktes mit Palladium oder seinen Verbindungen zu vermeiden, kann beispielsweise nach Ende der Reaktion der Reaktionsmischung soviel Komplexligand zugesetzt werden, daß das gesamte Palladium in Lösung bleibt.

Natürlich kann das Palladium auch gegebenenfalls durch chromatographische Methoden oder durch Ausfällen, beispielsweise als Sulfid, abgetrennt werden.

Der wasserlösliche, typischerweise phosphorhaltige Komplexligand wird durch Trennung von wäßriger und organischer Phase bereits vollständig vom Produkt abgetrennt.

Üblicherweise wird zur weiteren Aufarbeitung nach der Phasentrennung das Rohprodukt vom Lösungsmittel befreit und anschließend nach dem jeweiligen Produkt angemessenen Methoden, z.B. durch Umkristallisation, Destillation, Sublimation, Zonenschmelzen, Schmelzkristallisation oder Chromatographie, weiter aufgereinigt.

Die wäßrige Phase kann für erneute Kupplungsreaktionen eingesetzt werden, ohne daß ein Verlust an katalytischer Aktivität auftritt.

Für das erfindungsgemäße Verfahren geeignete organische Lösungsmittel, die in dem Reaktionsmedium eine organische Phase ausbilden, sind beispielsweise Ether, wie Diethylether, Dimethoxymethan, Diethylenglykoldimethylether, Diisopropylether, tert.-Butylmethylether, Kohlenwasserstoffe, wie Hexan, iso-Hexan, Heptan, Cyclohexan, Benzol, Toluol, Xylol, höhere, mit Wasser nicht beliebig mischbare Alkohole, wie 1-Butanol, 2-Butanol, tert.-Butanol, Amylalkohol, Ketone, z.B. iso-Butylmethylketon, Amide, wie Dimethylacetamid, N-Methylpyrrolidon, Nitrile, wie Butyronitril, und Mischungen derselben.

Bevorzugte organische Lösungsmittel sind Ether, wie Diethylether, Dimethoxyethan, Diethylenglykoldimethylether, Diisopropylether, Kohlenwasserstoffe, wie Hexan, Heptan, Cyclohexan, Benzol, Toluol, Xylol, Alkohole, wie 1-Butanol, 2-Butanol, tert.-Butanol, Ketone, wie iso-Butylmethylketon, Amide, wie N-Methylpyrrolidon, und Mischungen derselben.

Besonders bevorzugte organische Lösungsmittel sind cyclische oder acyclische, aliphatische oder aromatische Kohlenwasserstoffe, z.B. Cyclohexan, Benzol, Toluol, Xylol und Mischungen derselben.

In einer besonders bevorzugten Variante werden in dem erfindungsgemäßen Verfahren Wasser, ein oder mehrere in Wasser unlösliche und ein oder mehrere in Wasser lösliche Lösungsmittel eingesetzt.

Bevorzugte mit der wäßrigen Phase mischbare organische Cosolvenzien sind niedere Nitrile, wie Acetonitril, Formamide, wie DMF, niedere Alkohole, wie Methanol, Ethylenglykol und Ethanol, Sulfoxide, wie DMSO, und cyclische Ether, wie THF oder Dioxan.

Bevorzugte Reaktionsmedien aus Wasser, wasserlöslichem organischen Lösungsmittel und wasserunlöslichem organischen Lösungsmittel sind Mischungen aus Wasser, Toluol und Ethanol, Wasser, Xylol und Ethanol, Wasser, Toluol und Tetrahydrofuran, Wasser, Xylol und Tetrahydrofuran, Wasser, Xylol und Acetonitril und Wasser, Toluol und Acetonitril, vorzugsweise jeweils im Volumenverhältnis 1:2:1.

Basen, die bei dem erfindungsgemäßen Verfahren üblicherweise Verwendung finden, sind Alkali- und Erdalkalimetallhydroxide, Alkali- und Erdalkalimetallcarbonate, Alkalimetallhydrogencarbonate, Alkali- und Erdalkalimetallacetate, Alkali- und Erdalkalimetallalkoholate, sowie primäre, sekundäre und tertiäre Amine.

Besonders bevorzugt sind Alkali- und Erdalkalimetallhydroxide, Alkali- und Erdalkalimetallcarbonate und Alkalimetallhydrogencarbonate sowie sekundäre und tertiäre Alkylamine.
Insbesondere bevorzugt sind Alkalimetallhydroxide, wie Natriumhydroxid und Kaliumhydroxid, Alkalimetallcarbonate und Alkalimetallhydrogencarbonate, wie Lithiumcarbonat, Natriumcarbonat und Kaliumcarbonat, sowie sekundäre Amine wie Piperidin, Diethylamin oder Morpholin.

Die Base wird bei dem erfindungsgemäßen Verfahren bevorzugt mit einem Anteil von 50 bis 1000 Mol-%, besonders bevorzugt 65 bis 500 Mol-%, ganz besonders bevorzugt 75 bis 350 Mol-%, insbesondere 90 bis 200 Mol-%, bezogen auf die aromatische Halogenverbindung oder das Perfluoralkylsulfonat, eingesetzt.

Als Katalysator dienen zum einen in organischen Lösungsmitteln lösliche Palladiumverbindungen, wie Palladiumketonate, Palladiumacetylacetonate, Nitrilpalladiumhalogenide, Olefinpalladiumhalogenide, Palladiumhalogenide, Allylpalladiumhalogenide und Palladiumbiscarboxylate, bevorzugt Palladiumketonate, Palladiumacetylacetonate, bis-η²-Olefinpalladiumdihalogenide, Palladium(II)halogenide, η-³-Allylpalladiumhalogenid Dimere und Palladiumbiscarboxylate, ganz besonders bevorzugt Bis(dibenzylidenaceton)palladium(0) [Pd(dba)₂)], Pd(dba)₂·CHCl₃, Palladiumbisacetylacetonat, Bis(benzonitril)palladiumdichlorid, η³-Allylpalladiumchloriddimer, PdCl₂, Na₂PdCl₄, Dichlorobis(dimethylsulfoxid)palladium(II), Bis(acetonitril)palladiumdichlorid, Palladium-II-acetat, Palladium-II-propionat, Palladium-II-butanoat und (1c,5c-Cyclooctadien)palladiumdichlorid.

Zum anderen dienen Palladium oder Palladiumverbindungen in fester Form als Katalysator, vorzugsweise Palladium in pulverisierter Form oder auf einem Trägermaterial, z.B. Palladium auf Aktivkohle, Palladium auf Aluminumoxid, Palladium auf Bariumcarbonat, Palladium auf Bariumsulfat, Palladium auf Aluminiumsilikaten, wie Montmorillonit, Palladium auf SiO₂ und Palladium auf Calciumcarbonat, jeweils mit einem Palladiumgehalt von 0,5 bis 10 Gew.-%. Besonders bevorzugt sind Palladium in pulverisierter Form, Palladium auf Aluminiumoxid und Palladium auf Aktivkohle, jeweils mit einem Palladiumgehalt von 0,5 bis 10 Gew.-%. Insbesondere bevorzugt ist Palladium auf Aktivkohle mit einem Palladiumgehalt von 2 oder 7 Gew.-%. Es können auch Katalysatoren eingesetzt werden, die neben Palladium und dem Trägermaterial weitere Dotierstoffe, z.B. Blei (Lindlar-Katalysator), enthalten.

Der Palladiumkatalysator wird bei dem erfindungsgemäßen Verfahren mit einem Anteil von 0,001 bis 10 Mol-%, bevorzugt 0,01 bis 5 Mol-%, besonders bevorzugt 0,05 bis 3 Mol-%, insbesondere bevorzugt 0,1 bis 1,5 Mol-%, bezogen auf die aromatische Halogenverbindung oder das Perfluoralkylsulfonat, eingesetzt.

Weiterhin werden bei dem erfindungsgemäßen Verfahren vorteilhaft eine oder mehrere Kupfer(I)verbindungen als Cokatalysator eingesetzt.

Üblicherweise sind dies Kupfer(I)halogenide, wie CuI, CuBr, CuCl, Kupfer(I)cyanid, Kupfer(I)thiocyanat oder Kupfer(I)acetat, bevorzugt sind CuI und CuBr.

Die Kupfer(I)verbindung wird üblicherweise in einem stöchiometrischen Verhältnis von 0,5 bis 2:1, bezogen auf den Palladiumgehalt, eingesetzt. Vorzugsweise beträgt das stöchiometrische Verhältnis von Kupfer und Palladium 1:1.

Für das erfindungsgemäße Verfahren geeignete wasserlösliche Liganden enthalten beispielsweise Sulfonsäuresalz- und/oder Sulfonsäurereste und/oder Carbonsäuresalz- und/oder Carbonsäurereste und/oder Phosphonsäuresalz und/oder Phosphonsäurereste und/oder Phosphoniumgruppen und/oder Peralkylammoniumgruppen und/oder Hydroxygruppen und/oder Polyethergruppen mit geeigneter Kettenlänge.

Bevorzugte Klassen von wasserlöslichen Liganden sind mit den obigen Gruppen substituierte Phosphane, wie Trialkylphosphane, Tricycloalkylphosphane, Triarylphosphane, Dialkylarylphosphane, Alkyldiarylphosphane und Heteroarylphosphane, wie Tripyridylphosphan und Trifurylphosphan, wobei die drei Substituenten am Phosphor gleich oder verschieden, chiral oder achiral sein können und wobei einer oder mehrere der Liganden die Phosphorgruppen mehrerer Phosphane verknüpfen können und wobei ein Teil dieser Verknüpfung auch ein oder mehrere Metallatome sein können, Phosphite, Phosphinigsäureester und Phosphonigsäureester, Phosphole, Dibenzophosphole und Phosphoratome enthaltende cyclische oder oligo- und polycyclische Verbindungen.

Weitere geeignete Gruppen wasserlöslicher Komplexliganden umfassen beispielsweise Bipyridine, Phenanthroline, Porphyrine und Alizarine, die mit den obengenannten Gruppen modifiziert sind.

Bevorzugt eingesetzte wasserlösliche Phosphane sind solche der allgemeinen Formeln (I) bis (VII), 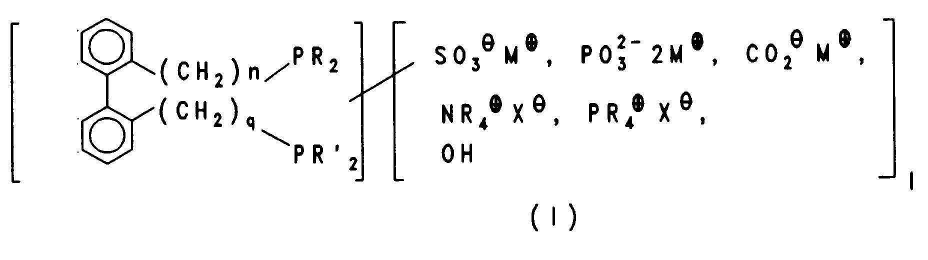 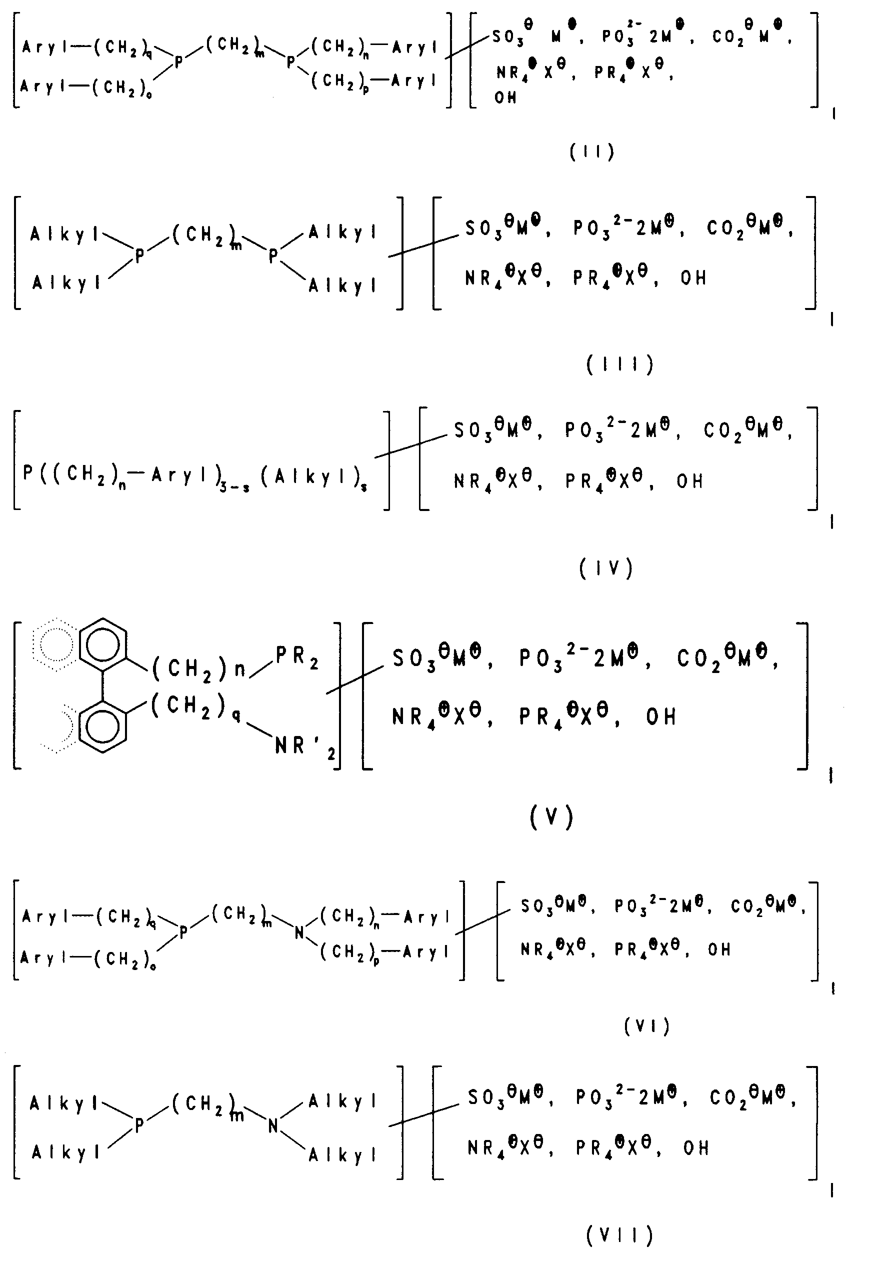
wobei die Symbole und Indizes folgende Bedeutungen haben:
- Aryl:: eine Phenyl- oder Naphthylgruppe, die auch einen oder mehrere Substituenten R tragen kann;
- Alkyl:: eine geradkettige oder verzweigte Alkylgruppe mit 1 bis 8 Kohlenstoffatomen;
- R,R':: Alkyl, Aryl oder Aralkyl mit 1 bis 18 Kohlenstoffatomen;
- M:: Alkali-, Erdalkalimetall oder NR₄;
- X:: Halogen, BF₄, PF₆, OSO₂CF₃, 1/2[SO₄];
- l,m:: 1 bis 8;
- n,o,p,q:: 0, 1 bis 8;
- s:: 0, 1 bis 3.

Im folgenden sind Beispiele für besonders bevorzugte wasserlösliche Komplexliganden aufgeführt:
(R hat dabei, wenn nicht anders vermerkt, die in den Formeln (I) bis (VII) angegebene Bedeutung)

### 1. Sulfonierte Phosphane

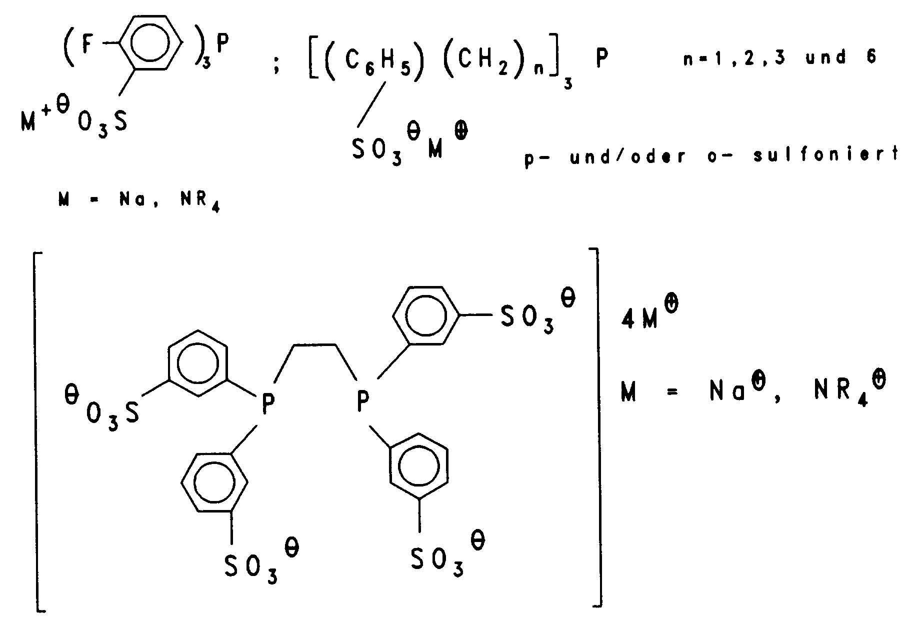

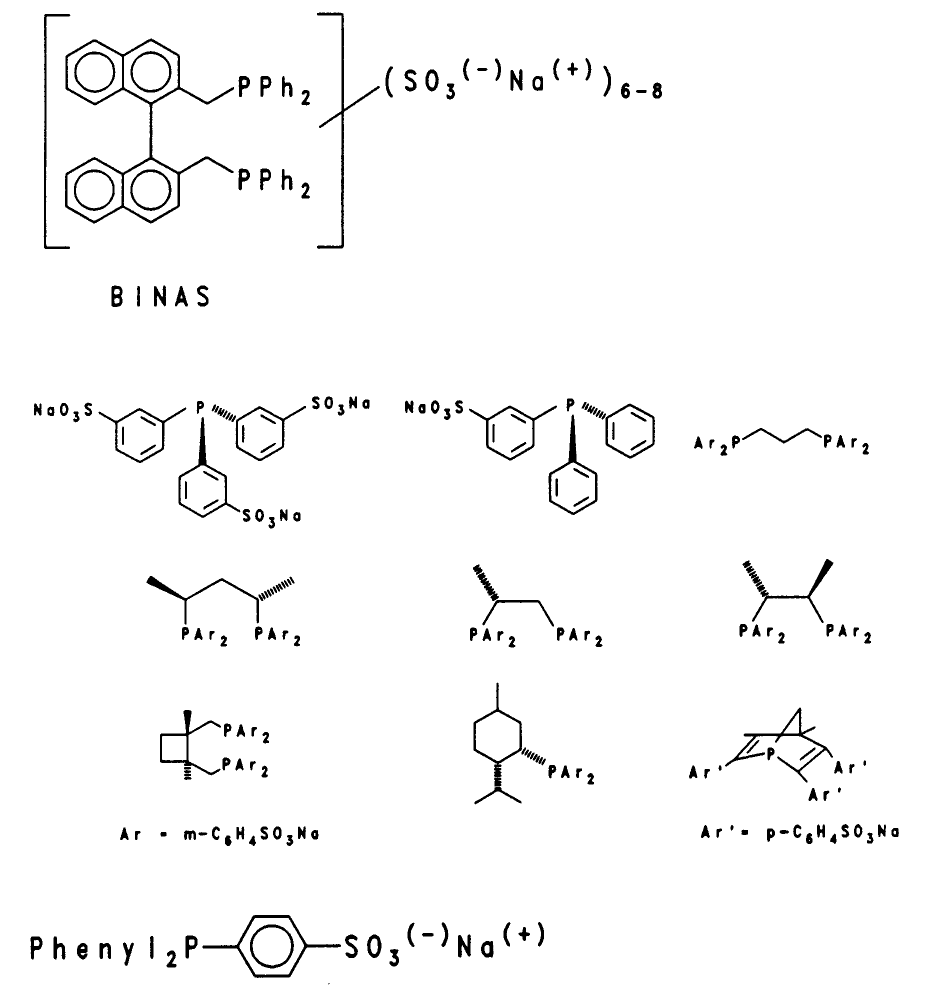

R₃₋ₙP(p-C₆H₄SO₃K)ₙ R = C₆H₅, 2-Pyridyl, 3-Pyridyl; n = 1-3 P[p-OC₆H₄SO₃(NH(i-octyl)₃]₃

### 1.1 Phosphane mit hydrophilen Gruppen in der Peripherie

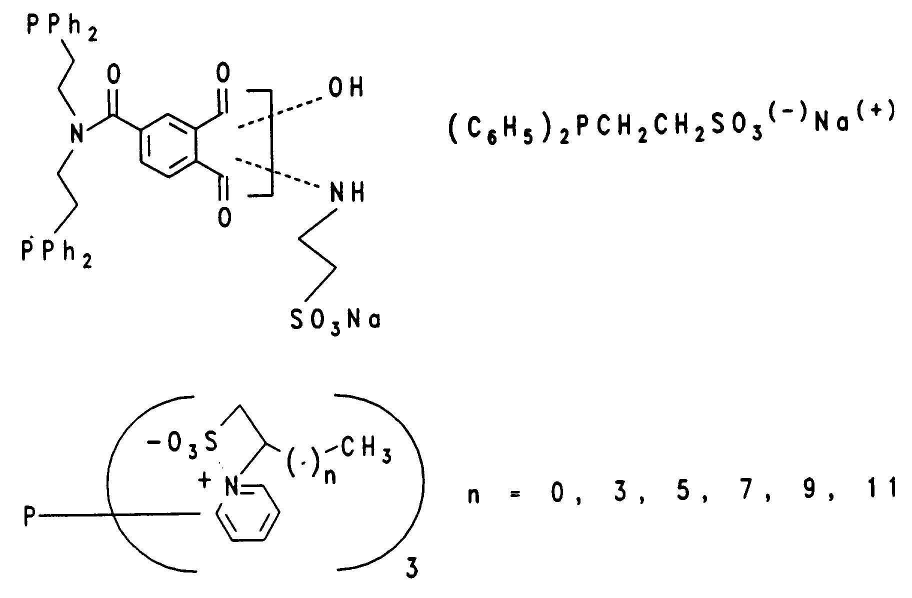

### 2. Phosphane mit quaternisierten Aminoalkyl- und Aminoaryl-Substituenten

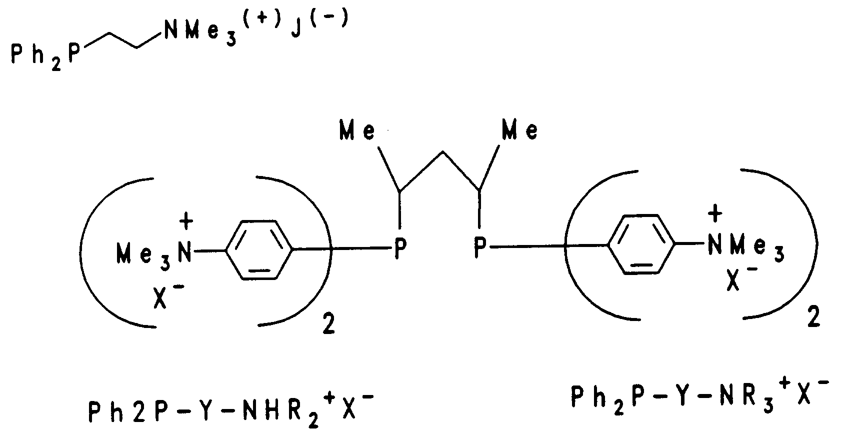

Y = -CH₂CH₂-, -CH(CH₃)CH₂-, -CH₂CH(CH₃)CH₂-; R = CH₃; X = J^{⊖}, Bu^{⊖}, Cl^{⊖}, OSO₂CF₃^{⊖}, BF₄^{⊖}, PF₆^{⊖}

### 3. Carboxylierte Phosphane

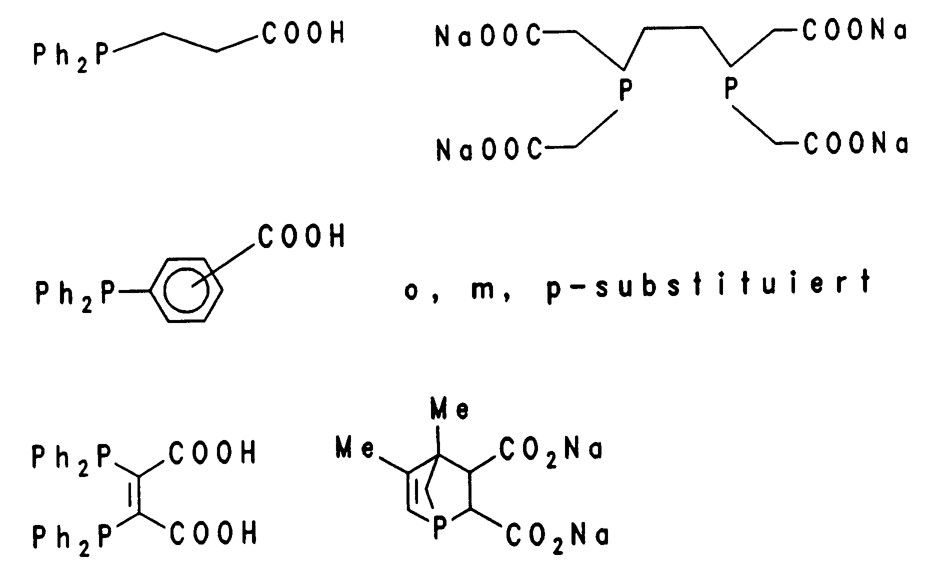

### 4. Phosphane mit Hydroxyalkyl- oder Polyether-Substituenten

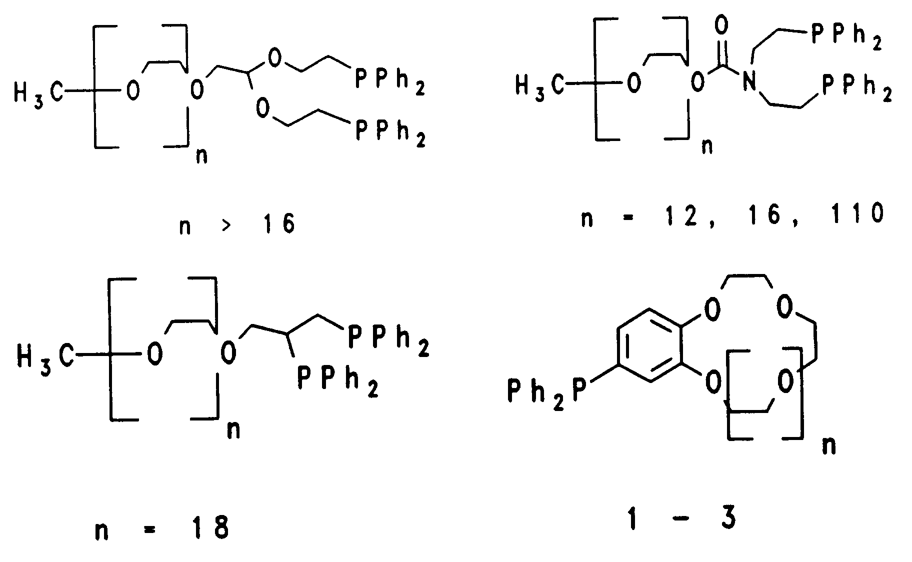

### 5. Phosphinoalkyl-phosphoniumsalze

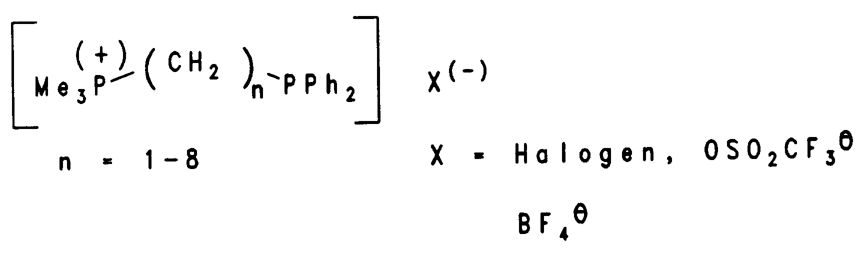

### 6. Phosphite

P[-OC₆H₄SO₃{NH(i-Octyl)₃}]₃

Insbesondere bevorzugte wasserlösliche Phosphanliganden sind: 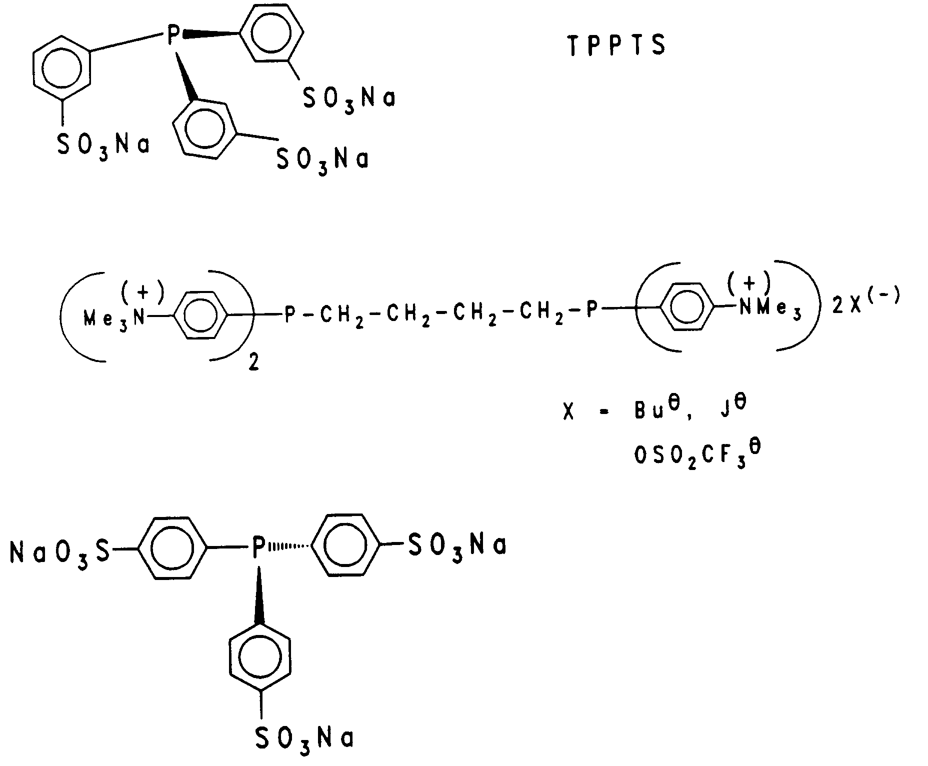 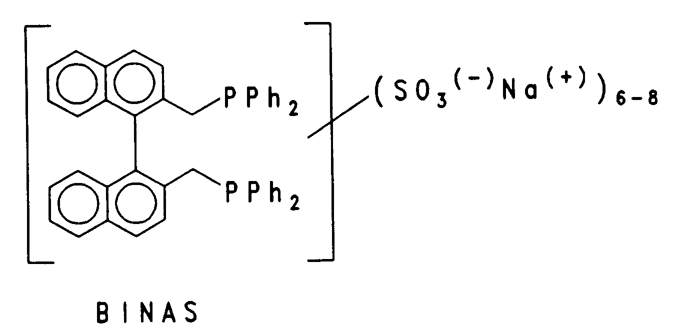
Der wasserlösliche Ligand enthält vorzugsweise 3 oder mehr die Wasserlöslichkeit bewirkende Gruppen. Vorzugsweise beträgt seine Löslichkeit in Wasser mindestens 20 mmol/l.

Der wasserlösliche Ligand wird bei dem erfindungsgemäßen Verfahren üblicherweise mit einem Anteil von 0,001 bis 20 Mol-%, bevorzugt 0,01 bis 15 Mol-%, besonders bevorzugt 0,05 bis 10 Mol-%, insbesondere bevorzugt 0,1 bis 6 Mol-%, bezogen auf die aromatische Halogenverbindung oder das Perfluoralkylsulfonat, eingesetzt.
Es können gegebenenfalls auch Mischungen zweier oder mehrerer verschiedener wasserlöslicher Komplexliganden eingesetzt werden.
Die erfindungsgemäß eingesetzten wasserlöslichen Komplexliganden sind zum großen Teil aus der Literatur bekannt. Die Synthesen dieser Verbindungen sind beispielsweise beschrieben bei W. A. Herrmann und C. W. Kohlpainter, Angew. Chem. Int. Ed. Engl. 1993, 32, 1524 und der dort zitierten Literatur oder können nach literaturbekannten oder analogen, dem Fachmann geläufigen Methoden erfolgen. Die Herstellung von BINAS ist in der EP-A 0 571819 bzw. US-A 5,347,045 beschrieben.

Ausgangsverbindungen für das erfindungsgemäße Verfahren sind zum einen terminale Alkine, d.h. Verbindungen die das Strukturelement -C≡C-H enthalten.

Neben der Alkinylgruppe enthalten die Verbindungen beispielsweise Alkyl-, Heteroalkyl-, Cycloalkyl-, Aryl-, Heteroaryl- und/oder Aralkylgruppen, die alle gegebenenfalls auch funktionalisiert sein können.

Beispiele für solche Verbindungen sind Acetylen, Phenylacetylen, Propargylalkohol, Propargyltrifluoracetamid, Propargylamin, 2-Methyl-3-butin-2-ol, Trimethylsilylacetylen und 1-Hexin.

Die zweite Klasse von Ausgangsverbindungen für das erfindungsgemäße Verfahren sind aromatische Halogenverbindungen oder aromatische Perfluoralkylsulfonate, vorzugsweise solche der Formel (VIII),

X-A³(-M²)ₘ(-A⁴)ₙ-R² (VIII),

wobei R², A³, A⁴, M², X m und n die folgenden Bedeutungen haben:
R² ist Benzyloxy, H, F, Cl, Br, -NC, -CN, -CF₃, -OCF₃, Isoxazolin oder ein geradkettiger, verzweigter (mit oder ohne asymmetrisches C-Atom) oder cyclischer Alkylrest mit 1 bis 18 C-Atomen, wobei auch eine oder zwei nicht benachbarte CH₂-Gruppen durch -O-, -S-, -CO-, -CO-O-, -O-CO-, -CO-S-, -S-CO-, -O-CO-O-, -SO₂-, -CON(H,C₁-C₈-Alkyl)-, -CH=CH-, -C≡C-, Cyclopropan-1,2-diyl oder -Si(CH₃)₂- ersetzt sein können, und wobei auch ein oder mehrere H-Atome des Alkylrestes durch F, Cl, Br oder CN substituiert sein können;,
A⁴ ist 1,4-Phenylen, Pyrazin-2,5-diyl, Pyridazin-3,6-diyl, Pyridin-2,5-diyl, Pyrimidin-2,5-diyl, trans-1,4-Cyclohexylen, Naphthalin-2,6-diyl, wobei bei jeder der vorhergehenden Gruppen ein oder mehrere H-Atome gleich oder verschieden durch Substituenten L ersetzt sein können, wobei L die unter R² genannten Bedeutungen hat, -CHO oder 4,4-Dimethylisoxazolin ist, und wobei eine oder zwei nicht benachbarte CH₂-Gruppen des Cyclohexylens durch -O- und/oder -S- ersetzt sein können, (1,3,4)-Thiadiazol-2,5-diyl, 1,3-Thiazol-2,4-diyl, 1,3-Thiazol-2,5-diyl, Thiophen-2,4-diyl, Thiophen-2,5-diyl, Piperazin-1,4-diyl, Piperazin-2,5-diyl, Piperidin-1,4-diyl, Bicyclo[2.2.2]octan-1,4-diyl, 1,3-Dioxaborinan-2,5-diyl oder trans-Dekalin-2,6-diyl;
A³ ist 1,4-Phenylen, Pyrazin-2,5-diyl, Pyridazin-3,6-diyl, Pyridin-2,5-diyl, Pyrimidin-2,5-diyl, Naphthalin-2,6-diyl, wobei bei jeder dieser Gruppen ein oder mehrere H-Atome gleich oder verschieden durch Substituenten L ersetzt sein können, wobei L die unter R² genannten Bedeutungen hat, -CHO oder 4,4-Dimethylisoxazolin ist, 1,3,4-Thiadiazol-2,5-diyl, 1,3-Thiazol-2,4-diyl, 1,3-Thiazol-2,5-diyl, Thiophen-2,4-diyl oder Thiophen-2,5-diyl;
M² ist
-O-, -S-, -CO-, -CO-O-, O-CO-, -CO-S, -S-CO-, -O-CO-O-, -CH₂-O-, -OCH₂-, -CH₂CH₂-, -CH=CH-, -C≡C-, -CH(CN)-CH₂-, -CH₂-CH(CN)-, -CH=N-, -N=CH-, -CH₂CH₂CH₂-O-, -OCH₂CH₂CH₂-, -CH₂CH₂CO-O-, -O-COCH₂CH₂-;
X ist Cl, Br, I oder ein Perfluoralkylsulfonat und
m, n bedeuten jeweils unabhängig voneinander Null oder Eins.

Bevorzugt bedeutet R² Benzyloxy, H, F, Cl, Br, -CN, -CF₃, -OCF₃ oder einen geradkettigen, verzweigten (mit oder ohne asymmetrisches C-Atom) oder cyclischen Alkylrest mit 1 bis 18 C-Atomen, wobei auch eine oder zwei nicht benachbarte CH₂-Gruppen durch -O-, -CO-, -CO-O-, -O-CO-, -O-CO-O-, -CH=CH-, -C≡C-, Cyclopropan-1,2-diyl oder -Si(CH₃)₂- ersetzt sein können, und wobei auch ein oder mehrere H-Atome des Alkylrestes durch F, Cl oder CN substituiert sein können.

Besonders bevorzugt bedeutet R² Benzyloxy, H, Cl, Br oder einen geradkettigen, verzweigten (mit oder ohne assymmetrisches C-Atom) oder cyclischen Alkylrest mit 1 bis 18 C-Atomen, wobei auch eine oder zwei nicht benachtbarte CH₂-Gruppen durch -O-, -CO-, -CO-O-, -O-, -O-CO-, -O-CO-O-, -CH=CH-, Cyclopropan-1,2-diyl oder -Si(CH₃)-ersetzt sein können.

Bevorzugt bedeutet A³ 1,4-Phenylen, Pyrazin-2,5-diyl, Pyridazin-3,6-diyl, Pyridin-2,5-diyl, Pyrimidin-2,5-diyl, Naphthalin-2,6-diyl, wobei bei jeder dieser Gruppen ein oder mehrere H-Atome gleich oder verschieden durch Substituenten L ersetzt sein können, wobei L die unter R² genannten Bedeutungen hat, -CHO oder 4,4-Dimethylisoxazolin ist, oder 1,3,4-Thiadiazol-2,5-diyl.

Besonders bevorzugt bedeutet A³ 1,4-Phenylen, 2-Fluor-1,4-phenylen, 2,3-Difluor-1,4-phenylen, 2,6-Difluor-1,4-phenylen, 2,5-Difluor-1,4-phenylen, sowie Pyrazin-2,5-diyl, Pyridazin-2,5-diyl, Pyrimidin-2,5-diyl oder Naphthalin-2,6-diyl, wobei bei jeder dieser Gruppen ein oder mehrere H-Atome gleich oder verschieden durch Substituenten L ersetzt sein können, wobei L die unter R² genannten Bedeutungen hat, -CHO oder 4,4-Dmethylisoxazolin ist.

Bevorzugt bedeutet A⁴ 1,4-Phenylen, Pyrazin-2,5-diyl, Pyridazin-3,6-diyl, Pyridin-2,5-diyl, Pyrimidin-2,5-diyl, trans-1,4-Cyclohexylen, Naphthalin-2,6-diyl, wobei bei jeder dieser Gruppen ein oder mehrere H-Atome gleich oder verschieden durch Substituenten L ersetzt sein können, wobei L die unter R² genannten Bedeutungen hat, und wobei eine oder zwei nicht benachbarte CH₂-Gruppen des Cyclohexylens durch -O- ersetzt sein können, (1,3,4)-Thiadiazol-2,5-diyl oder Bicyclo[2.2.2]octan-1,4-diyl.

Besonders bevorzugt bedeutet A⁴ 1,4-Phenylen, 2-Fluor-1,4-phenylen, 2,3-Difluor-1,4-phenylen, 2,6-Difluor-1,4-phenylen, 2,5-Difluor-1,4-phenylen, sowie Pyridin-2,5-diyl, Pyrimidin-2,5-diyl, trans-1,4-Cyclohexylen, Naphthalin-2,6-diyl, wobei bei jeder dieser Gruppen ein oder mehrere H-Atome gleich oder verschieden durch Substituenten L ersetzt sein können, wobei L die unter R² genannten Bedeutungen hat.

Bevorzugt bedeutet M² -O-, -CO-, -CO-O-, -O-CO-, -O-CO-O-, -CH₂-O-, -O-CH₂-, -CH₂CH₂-, -CH=CH-, -C≡C-, -CH₂CH₂CH₂-O-, -O-CH₂CH₂CH₂-, -CH₂CH₂CO-O- oder -O-CO-CH₂CH₂-.

Besonders bevorzugt bedeutet M² -O-, -CO-, -CO-O-, -O-CO-, -O-CO-O-, -CH₂-O-, -O-CH₂-, -CH₂CH₂-, -CH=CH-, -C≡C-, -CH₂CH₂CH₂-O-, -O-CH₂CH₂CH₂-, -CH₂CH₂CO-O- oder -O-CO-CH₂CH₂.

Bevorzugt bedeutet X Brom, Iod oder OSO₂-CₚF_{2p + 1}, worin p einen ganzzahligen Wert von 1 bis 10 darstellt. Besonders bevorzugt bedeutet X Brom.

Die verwendeten aromatischen Halogenverbindungen und Perfluoralkylsulfonate sind entweder bekannt oder können nach bekannten Methoden, wie beispielsweise in Houben Weyl, Methoden der Organischen Chemie, Georg Thieme Verlag, Stuttgart, Band 5/3 und 5/4 beschrieben, hergestellt werden. Beispielsweise lassen sich aromatische Halogenide dadurch erhalten, daß man in einem entsprechenden Diazoniumsalz die Diazoniumgruppe durch Chlor, Brom oder Iod ersetzt.
Desweiteren lassen sich Hydroxy-Stickstoffheterocyclen mit Hilfe von Phosphortrihalogeniden und Phosphoroxytrihalogeniden in die entsprechenden Halogenide überführen.

Die Produkte des erfindungsgemäßen Verfahrens sind 1-Arylalkine, d.h. Verbindungen, die das Strukturelement -Ar-C≡C- (wobei Ar für eine Aryl- oder Heteroarylgruppe steht) enthalten.

Solche Verbindungen eignen sich unter anderem als flüssigkristalline Materialien oder können als Zwischenprodukte für die Herstellung von flüssigkristallinen Verbindungen dienen. Desweiteren finden sie Anwendungen als Vorprodukte für Pharmazeutika, Kosmetika, Fungizide, Herbizide, Insektizide, Farbstoffe, Detergenzien und Polymere, einschließlich von Zusatzstoffen derselben.

Die vorliegende Erfindung soll durch die nachfolgend beschriebenen Beispiele näher erläutert werden, ohne sie dadurch zu begrenzen. Die verwendeten Abkürzungen haben dabei folgende Bedeutung:
- Fp.: = Schmelzpunkt
- X: = kristallin
- S: = smektisch
- S_{C}: = smektisch C
- S_{A}: = smektisch A
- N: = nematisch
- I: = isotrop

### TPPTS:

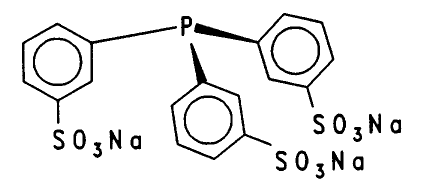

### BINAS:

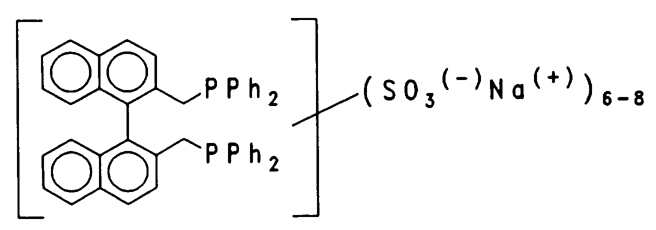

### Beispiel 1:

9,5 g 1,4-Chloriodbenzol (0,04 mol) werden unter Inertgas in 50 ml Toluol gelöst. Man versetzt mit 25 ml Wasser und 25 ml Ethanol. Nacheinander gibt man 3,5 ml (0,06 mol) Propargylalkohol, 2 ml einer 0,6 millimolaren wäßrigen TPPTS-Lösung und 0,09 g (0,4 mmol) Palladium(II)acetat, gelöst in 5 ml Toluol, zu. Die Mischung wird 15 Minuten gerührt. Die Reaktionsmischung wird auf 0°C gekühlt. Anschließend gibt man 5,1 g (0,06 mol) Piperidin und 11,4 mg (0,6 mmol) Kupfer(I)iodid zu.

Die Reaktionsmischung wird 1 Stunde bei 0°C und 15 Stunden bei Raumtemperatur gerührt. Anschließend versetzt man mit 100 ml gesättigter wäßriger Ammoniumchloridlösung und 100 ml Ethylacetat. Die organische Phase wird abgetrennt und über Natriumsulfat getrocknet. Nach Einengen der organischen Phase wird das Rohprodukt aus 200 ml Methylcyclohexan bei -20°C umkristallisiert.
Ausbeute: 5,4 g (0,0325 mol; 81 % d.Th.); Schmelzpunkt: 74°C

### Beispiel 2:

9,5 g 1,4-Chloriodbenzol (0,04 mol) werden unter Inertgas in 50 ml Toluol gelöst. Man versetzt mit 25 ml Wasser und 25 ml Ethanol. Nacheinander gibt man 3,5 ml (0,06 mol) Propargylalkohol, 2 ml einer 0,6 millimolaren wäßrigen TPPTS-Lösung und 0,07 g (0,4 mmol) Palladium(II)chlorid, gelöst in 5 ml Toluol, zu. Die Mischung wird 15 Minuten gerührt. Die Reaktionsmischung wird auf 0°C gekühlt. Anschließend gibt man 5,1 g (0,06 mol) Piperidin und 11,4 mg (0,6 mmol) Kupfer(I)iodid zu.

Die Reaktionsmischung wird 1 Stunden bei 0°C und 15 Stunden bei Raumtemperatur gerührt. Anschließend versetzt man mit 100 ml gesättigter wäßriger Ammoniumchloridlösung und 100 ml Ethylacetat. Die organische Phase wird abgetrennt und über Natriumsulfat getrocknet. Nach Einengen der organischen Phase wird das Rohprodukt aus 200 ml Methylcyclohexan bei -20°C umkristallisiert.
Ausbeute: 5,6 g (0,0337 mol; 84 % d.Th.); Schmelzpunkt: 74°C

### Beispiel 3:

9,5 g 1,4-Chloriodbenzol (0,04 mol) werden unter Inertgas in 50 ml Toluol gelöst. Man versetzt mit 25 ml Wasser und 25 ml Ethanol. Nacheinander gibt man 3,5 ml (0,06 mol) Propargylalkohol, 2 ml einer 0,6 millimolaren wäßrigen TPPTS-Lösung und 0,09 g (0,4 mmol) Palladium(II)acetat, gelöst in 5 ml Toluol, zu. Die Mischung wird 15 Minuten gerührt. Die Reaktionsmischung wird auf 0°C gekühlt. Anschließend gibt man 7,5 g (0,06 mol) 32 gew.-%ige Natronlauge und 11,4 mg (0,6 mmol) Kupfer(I)iodid zu.

Die Reaktionsmischung wird 1 Stunde bei 0°C und 15 Stunden bei Raumtemperatur gerührt. Anschließend versetzt man mit 100 ml gesättigter wäßriger Ammoniumchloridlösung und 100 ml Ethylacetat. Die organische Phase wird abgetrennt und über Natriumsulfat getrocknet. Nach Einengen der organischen Phase wird das Rohprodukt aus 200 ml Methylcyclohexan bei -20°C umkristallisiert.
Ausbeute: 5,8 g (0,0349 mol; 87 % d.Th.); Schmelzpunkt: 74°C

### Beispiel 4:

9,5 g 1,4-Chloriodbenzol (0,04 mol) werden unter Inertgas in 50 ml Toluol gelöst. Man versetzt mit 25 ml Wasser und 25 ml Ethanol. Nacheinander gibt man 3,5 ml (0,06 mol) Propargylalkohol, 2 ml einer 0,6 millimolaren wäßrigen TPPTS-Lösung und 0,07 g (0,4 mmol) Palladium(II)chlorid, gelöst in 5 ml Toluol, zu. Die Mischung wird 15 Minuten gerührt. Die Reaktionsmischung wird auf 0°C gekühlt. Anschließend gibt man 7,5 g (0,06 mol) 32 gew.-%ige Natronlauge und 11,4 mg (0,6 mmol) Kupfer(I)iodid zu.

Die Reaktionsmischung wird 1 Stunde bei 0°C und 15 Stunden bei Raumtemperatur gerührt. Anschließend versetzt man mit 100 ml gesättigter wäßriger Ammoniumchloridlösung und 100 ml Ethylacetat. Die organische Phase wird abgetrennt und über Natriumsulfat getrocknet. Nach Einengen der organischen Phase wird das Rohprodukt aus 200 ml Methylcyclohexan bei -20°C umkristallisiert.
Ausbeute: 5,8 g (0,0349 mol; 87 % d.Th.); Schmelzpunkt: 74°C

### Beispiel 5:

9,5 g 1,4-Chloriodbenzol (0,04 mol) werden unter Inertgas in 50 ml Toluol gelöst. Man versetzt mit 25 ml Wasser und 25 ml Ethanol. Nacheinander gibt man 3,5 ml (0,06 mol) Propargylalkohol, 2 ml einer 0,6 millimolaren wäßrigen TPPTS-Lösung und 0,09 g (0,4 mmol) Palladium(II)acetat, gelöst in 5 ml Toluol, zu. Die Mischung wird 15 Minuten gerührt. Die Reaktionsmischung wird auf 0°C gekühlt. Anschließend gibt man 6,24 g (0,06 mol) Natriumcarbonat, gelöst in 10 ml Wasser und 11,4 mg (0,6 mmol) Kupfer(I)iodid zu.

Die Reaktionsmischung wird 1 Stunde bei 0°C und 15 Stunden bei Raumtemperatur gerührt. Anschließend versetzt man mit 100 ml gesättigter wäßriger Ammoniumchloridlösung und 100 ml Ethylacetat. Die organische Phase wird abgetrennt und über Natriumsulfat getrocknet. Nach Einengen der organischen Phase wird das Rohprodukt aus 200 ml Methylcyclohexan bei -20°C umkristallisiert.
Ausbeute: 5,2 g (0,0313 mol; 78 % d.Th.); Schmelzpunkt: 74°C

### Beispiel 6:

9,5 g 1,4-Chloriodbenzol (0,04 mol) werden unter Inertgas in 50 ml Toluol gelöst. Man versetzt mit 25 ml Wasser und 25 ml Ethanol. Nacheinander gibt man 3,5 ml (0,06 mol) Propargylalkohol, 2 ml einer 0,6 millimolaren wäßrigen TPPTS-Lösung und 0,07 g (0,4 mmol) Palladium(II)chlorid, gelöst in 5 ml Toluol, zu. Die Mischung wird 15 Minuten gerührt. Die Reaktionsmischung wird auf 0°C gekühlt. Anschließend gibt man 6,24 g (0,06 mol) Natriumcarbonat, gelöst in 10 ml Wasser und 11,4 mg (0,6 mmol) Kupfer(I)iodid zu.

Die Reaktionsmischung wird 1 Stunde bei 0°C und 15 Stunden bei Raumtemperatur gerührt. Anschließend versetzt man mit 100 ml gesättigter wäßriger Ammoniumchloridlösung und 100 ml Ethylacetat. Die organische Phase wird abgetrennt und über Natriumsulfat getrocknet. Nach Einengen der organischen Phase wird das Rohprodukt aus 200 ml Methylcyclohexan bei -20°C umkristallisiert.
Ausbeute: 5,3 g (0,0319 mol; 80 % d.Th.); Schmelzpunkt: 74°C

### Beispiel 7:

9,5 g 1,4-Chloriodbenzol (0,04 mol) werden unter Inertgas in 50 ml Toluol gelöst. Man versetzt mit 25 ml Wasser und 25 ml Ethanol. Nacheinander gibt man 3,5 ml (0,06 mol) Propargylalkohol, 2 ml einer 0,6 millimolaren wäßrigen TPPTS-Lösung und 0,426 g (0,4 mmol) Palladium auf Aktivkohle (10 Gew.-%), suspendiert in 5 ml Toluol, zu. Die Mischung wird 15 Minuten gerührt. Die Reaktionsmischung wird auf 0°C gekühlt. Anschließend gibt man 5,1 g (0,06 mol) Piperidin und 11,4 mg (0,6 mmol) Kupfer(I)iodid zu.

Die Reaktionsmischung wird 1 Stunde bei 0°C und 15 Stunden bei Raumtemperatur gerührt. Anschließend versetzt man mit 100 ml gesättigter wäßriger Ammoniumchloridlösung und 100 ml Ethylacetat. Die organische Phase wird abgetrennt und über Natriumsulfat getrocknet. Nach Einengen der organischen Phase wird das Rohprodukt aus 200 ml Methylcyclohexan bei -20°C umkristallisiert.
Ausbeute: 6,1 g (0,0367 mol; 92 % d.Th.); Schmelzpunkt: 74°C

### Beispiel 8:

Unter Schutzgas werden 237,1 g (1,0 mol) 2-Brom-6-methoxynaphthalin und 126,2 g (1,5 mol) 2-Methyl-3-butin-2-ol zu einem Gemisch aus 1250 ml Toluol, 625 ml Ethanol und 625 ml Wasser gegeben. Man versetzt mit 50 ml 0,6 millimolarer wäßriger TPPTS-Lösung und rührt 5 Minuten. 2,25 g (10 mmol) Palladium(II)acetat werden in 15 ml Toluol gelöst und dem Reaktionsgemisch zugefügt. Man rührt weitere 15 Minuten bei Raumtemperatur. Innerhalb von 30 Minuten wird mit 109,7 g (1,5 mol) Diethylamin tropfenweise versetzt. Man rührt 10 Minuten nach. 3,8 g (20 mmol) Kupfer(I)iodid werden in 20 ml Acetonitril suspendiert und zur Reaktionslösung gegeben. Man rührt 17 Stunden unter Rückfluß. Zur Aufarbeitung gießt man auf eine Lösung aus 500 g Ammoniumchlorid in 2 Liter Wasser. Es wird mit Ethylacetat extrahiert, mit gesättigter wäßriger NaCl-Lösung gewaschen, über MgSO₄ getrocknet und das Solvens im Vakuum abgezogen. Die Umsetzung ist quantitativ. Nach Umkristallisation erhält man 191,8 g (798 mmol) 4-(6-Methoxy-2-naphthyl)-2-methyl-3-butin-2-ol.

### Beispiel 9:

Unter Schutzgas werden 213,1 g (1,0 mol) 4-Brom-1-isobutylbenzol und 126,2 g (1,5 mol) 2-Methyl-3-butin-2-ol zu einem Gemisch aus 1250 ml Toluol, 625 ml Ethanol und 625 ml Wasser gegeben. Man versetzt mit 50 ml 0,6 millimolarer wäßriger TPPTS-Lösung und rührt 5 Minuten. 2,25 g (10 mmol) Palladium(II)acetat werden in 15 ml Toluol gelöst und dem Reaktionsgemisch zugefügt. Man rührt weitere 15 Minuten bei Raumtemperatur. Innerhalb von 30 Minuten wird mit 109,7 g (1,5 mol) Diethylamin tropfenweise versetzt. Man rührt 10 Minuten nach. 3,8 g (20 mmol) Kupfer(I)iodid werden in 20 ml Acetonitril suspendiert und zur Reaktionslösung gegeben. Man rührt 17 Stunden unter Rückfluß. Zur Aufarbeitung gießt man auf eine Lösung aus 500 g Ammoniumchlorid in 2 Liter Wasser. Es wird mit Ethylacetat extrahiert, mit gesättigter wäßriger NaCl-Lösung gewaschen, über MgSO₄ getrocknet und das Solvens im Vakuum abgezogen. Man erhält 175,7 g (812 mmol) 4-(4-Isobutyl-1-phenyl)-2-methyl-3-butin-2-ol.

### Beispiel 10:

Unter Schutzgas werden 349,3 g (1,0 mol) 5-Brom-2-(4-octyloxyphenyl)pyrimidin und 165,3 g (1,5 mol) 1-Octin zu einem Gemisch aus 1250 ml Toluol, 625 ml Ethanol und 625 ml Wasser gegeben. Man versetzt mit 50 ml 0,6 millimolarer wäßriger TPPTS-Lösung und rührt 5 Minuten. 2,25 g (10 mmol) Palladium(II)acetat werden in 15 ml Toluol gelöst und dem Reaktionsgemisch zugefügt. Man rührt weitere 15 Minuten bei Raumtemperatur. Innerhalb von 30 Minuten wird mit 109,7 g (1,5 mol) Diethylamin tropfenweise versetzt. Man rührt 10 Minuten nach. 3,8 g (20 mmol) Kupfer(I)iodid werden in 20 ml Acetonitril suspendiert und zur Reaktionslösung gegeben. Man rührt 17 Stunden unter Rückfluß. Zur Aufarbeitung gießt man auf eine Lösung aus 500 g Ammoniumchlorid in 2 Liter Wasser. Es wird mit Ethylacetat extrahiert, mit gesättigter wäßriger NaCl-Lösung gewaschen, über MgSO₄ getrocknet und das Solvens im Vakuum abgezogen. Man erhält 5-(1-Octinyl)-2-(4-octyloxyphenyl)pyrimidin. Das Rohprodukt wird an Palladium/Kohle zu 5-Octyl-2-(4-octyloxyphenyl)pyrimidin hydriert.
- Phasen:: X 29(25) S_{c} 55 Sₐ 62 N 69 I

### Beispiel 11:

Unter Schutzgas werden 321,3 g (1,0 mol) 5-Brom-2-(4-hexyloxyphenyl)pyrimidin und 123,2 g (1,5 mol) 1-Hexin zu einem Gemisch aus 1250 ml Toluol, 625 ml Ethanol und 625 ml Wasser gegeben. Man versetzt mit 50 ml 0,6 millimolarer wäßriger TPPTS-Lösung und rührt 5 Minuten. 2,25 g (10 mmol) Palladium(II)acetat werden in 15 ml Toluol gelöst und dem Reaktionsgemisch zugefügt. Man rührt weitere 15 Minuten bei Raumtemperatur. Innerhalb von 30 Minuten wird mit 109,7 g (1,5 mol) Diethylamin tropfenweise versetzt. Man rührt 10 Minuten nach. 3,8 g (20 mmol) Kupfer(I)iodid werden in 20 ml Acetonitril suspendiert und zur Reaktionslösung gegeben. Man rührt 17 Stunden unter Rückfluß. Zur Aufarbeitung gießt man auf eine Lösung aus 500 g Ammoniumchlorid in 2 Liter Wasser. Es wird mit Ethylacetat extrahiert, mit gesättigter wäßriger NaCl-Lösung gewaschen, über MgSO₄ getrocknet und das Solvens im Vakuum abgezogen. Man erhält 5-(1-Hexinyl)-2-(4-hexyloxyphenyl)pyrimidin. Das Rohprodukt wird an Palladium/Kohle zu 5-Hexyl-2-(4-hexyloxyphenyl)pyrimidin hydriert.
- Phasen:: X₁ 15 X₂ 32 N 61 I

### Beispiel 12:

Unter Schutzgas werden 321,3 g (1,0 mol) 5-Brom-2-(4-hexyloxyphenyl)pyrimidin und 207,4 g (1,5 mol) 1-Decin zu einem Gemisch aus 1250 ml Toluol, 625 ml Ethanol und 625 ml Wasser gegeben. Man versetzt mit 50 ml 0,6 millimolarer wäßriger TPPTS-Lösung und rührt 5 Minuten. 2,25 g (10 mmol) Palladium(II)acetat werden in 15 ml Toluol gelöst und zum Reaktionsgemisch zugefügt. Man rührt weitere 15 Minuten bei Raumtemperatur. Innerhalb von 30 Minuten wird mit 109,7 g (1,5 mol) Diethylamin tropfenweise versetzt. Man rührt 10 Minuten nach. 3,8 g (20 mmol) Kupfer(I)iodid werden in 20 ml Acetonitril suspendiert und zur Reaktionslösung gegeben. Man rührt 17 Stunden unter Rückfluß. Zur Aufarbeitung gießt man auf eine Lösung aus 500 g Ammoniumchlorid in 2 Liter Wasser. Es wird mit Ethylacetat extrahiert, mit gesättigter wäßriger NaCl-Lösung gewaschen, über MgSO₄ getrocknet und das Solvens im Vakuum abgezogen. Man erhält 5-(1-Decinyl)-2-(4-hexyloxyphenyl)pyrimidin. Das Rohprodukt wird an Palladium/Kohle zu 5-Decyl-2-(4-hexyloxyphenyl)pyrimidin hydriert.
- Phasen:: X₁ 36 S_{c} 60 Sₐ 71 I

### Beispiel 13:

Unter Schutzgas werden 349,3 g (1,0 mol) 5-Brom-2-(4-octyloxyphenyl)pyrimidin und 207,4 g (1,5 mol) 1-Decin zu einem Gemisch aus 1250 ml Toluol, 625 ml Ethanol und 625 ml Wasser gegeben. Man versetzt mit 50 ml 0,6 millimolarer wäßriger TPPTS-Lösung und rührt 5 Minuten. 2,25 g (10 mmol) Palladium(II)acetat werden in 15 ml Toluol gelöst und dem Reaktionsgemisch zugefügt. Man rührt weitere 15 Minuten bei Raumtemperatur. Innerhalb von 30 Minuten wird mit 109,7 g (1,5 mol) Diethylamin tropfenweise versetzt. Man rührt 10 Minuten nach. 3,8 g (20 mmol) Kupfer(I)iodid werden in 20 ml Acetonitril suspendiert und zur Reaktionslösung gegeben. Man rührt 17 Stunden unter Rückfluß. Zur Aufarbeitung gießt man auf eine Lösung aus 500 g Ammoniumchlorid in 2 Liter Wasser. Es wird mit Ethylacetat extrahiert, mit gesättigter wäßriger NaCl-Lösung gewaschen, über MgSO₄ getrocknet und das Solvens im Vakuum abgezogen. Man erhält 5-(1-Decinyl)-2-(4-octyloxyphenyl)pyrimidin. Das Rohprodukt wird an Palladium/Kohle zu 5-Decyl-2-(4-octyloxyphenyl)pyrimidin hydriert.
- Phasen:: X₁ 40(30) S_{c} 69 Sₐ 74 I

### Beispiel 14:

9,5 g 1,4-Chloriodbenzol (0,04 mol) werden unter Inertgas in 50 ml Toluol gelöst. Man versetzt mit 25 ml Wasser und 25 ml Ethanol. Nacheinander gibt man 3,5 ml (0,06 mol) Propargylalkohol, 0,4 mmol BINAS in Form einer wäßrigen Lösung und 0,09 g (0,4 mmol) Palladium(II)acetat, gelöst in 5 ml Toluol, zu. Die Mischung wird 15 Minuten gerührt. Die Reaktionsmischung wird auf 0°C gekühlt. Anschließend gibt man 5,1 g (0,06 mol) Piperidin und 11,4 mg (0,6 mmol) Kupfer(I)iodid zu.

Die Reaktionsmischung wird 1 Stunde bei 0°C und 15 Stunden bei Raumtemperatur gerührt. Anschließend versetzt man mit 100 ml gesättigter wäßriger Ammoniumchloridlösung und 100 ml Ethylacetat. Die organische Phase wird abgetrennt und über Natriumsulfat getrocknet. Nach Einengen der organischen Phase wird das Rohprodukt aus 200 ml Methylcyclohexan bei -20°C umkristallisiert.
Ausbeute: 5,4 g (0,0325 mol; 81 % d.Th.); Schmelzpunkt: 74°C

### Beispiel 15:

9,5 g 1,4-Chloriodbenzol (0,04 mol) werden unter Inertgas in 50 ml Toluol gelöst. Man versetzt mit 25 ml Wasser und 25 ml Ethanol. Nacheinander gibt man 3,5 ml (0,06 mol) Propargylalkohol, 0,4 mmol BINAS in Form einer wäßrigen Lösung und 0,07 g (0,4 mmol) Palladium(II)chlorid, gelöst in 5 ml Toluol, zu. Die Mischung wird 15 Minuten gerührt. Die Reaktionsmischung wird auf 0°C gekühlt. Anschließend gibt man 5,1 g (0,06 mol) Piperidin und 11,4 mg (0,6 mmol) Kupfer(I)iodid zu.

Die Reaktionsmischung wird 1 Stunden bei 0°C und 15 Stunden bei Raumtemperatur gerührt. Anschließend versetzt man mit 100 ml gesättigter wäßriger Ammoniumchloridlösung und 100 ml Ethylacetat. Die organische Phase wird abgetrennt und über Natriumsulfat getrocknet. Nach Einengen der organischen Phase wird das Rohprodukt aus 200 ml Methylcyclohexan bei -20°C umkristallisiert.
Ausbeute: 5,6 g (0,0337 mol; 84 % d.Th.); Schmelzpunkt: 74°C

### Beispiel 16:

9,5 g 1,4-Chloriodbenzol (0,04 mol) werden unter Inertgas in 50 ml Toluol gelöst. Man versetzt mit 25 ml Wasser und 25 ml Ethanol. Nacheinander gibt man 3,5 ml (0,06 mol) Propargylalkohol, 0,4 mmol BINAS in Form einer wäßrigen Lösung und 0,09 g (0,4 mmol) Palladium(II)acetat, gelöst in 5 ml Toluol, zu. Die Mischung wird 15 Minuten gerührt. Die Reaktionsmischung wird auf 0°C gekühlt. Anschließend gibt man 7,5 g (0,06 mol) 32 gew.-%ige Natronlauge und 11,4 mg (0,6 mmol) Kupfer(I)iodid zu.

Die Reaktionsmischung wird 1 Stunde bei 0°C und 15 Stunden bei Raumtemperatur gerührt. Anschließend versetzt man mit 100 ml gesättigter wäßriger Ammoniumchloridlösung und 100 ml Ethylacetat. Die organische Phase wird abgetrennt und über Natriumsulfat getrocknet. Nach Einengen der organischen Phase wird das Rohprodukt aus 200 ml Methylcyclohexan bei -20°C umkristallisiert.
Ausbeute: 5,8 g (0,0349 mol; 87 % d.Th.); Schmelzpunkt: 74°C

### Beispiel 17:

9,5 g 1,4-Chloriodbenzol (0,04 mol) werden unter Inertgasbedingungen in 50 ml Toluol gelöst. Man versetzt mit 25 ml Wasser und 25 ml Ethanol. Nacheinander gibt man 3,5 ml (0,06 mol) Propargylalkohol, 0,4 mmol BINAS in Form einer wäßrigen Lösung und 0,07 g (0,4 mmol) Palladium(II)chlorid, gelöst in 5 ml Toluol, zu. Die Mischung wird 15 Minuten gerührt. Die Reaktionsmischung wird auf 0°C gekühlt. Anschließend gibt man 7,5 g (0,06 mol) 32 gew.-%ige Natronlauge und 11,4 mg (0,6 mmol) Kupfer(I)iodid zu.

Die Reaktionsmischung wird 1 Stunde bei 0°C und 15 Stunden bei Raumtemperatur gerührt. Anschließend versetzt man mit 100 ml gesättigter wäßriger Ammoniumchloridlösung und 100 ml Ethylacetat. Die organische Phase wird abgetrennt und über Natriumsulfat getrocknet. Nach Einengen der organischen Phase wird das Rohprodukt aus 200 ml Methylcyclohexan bei -20°C umkristallisiert.
Ausbeute: 5,8 g (0,0349 mol; 87 % d.Th.); Schmelzpunkt: 74°C

### Beispiel 18:

9,5 g 1,4-Chloriodbenzol (0,04 mol) werden unter Inertgas in 50 ml Toluol gelöst. Man versetzt mit 25 ml Wasser und 25 ml Ethanol. Nacheinander gibt man 3,5 ml (0,06 mol) Propargylalkohol, 0,4 mmol BINAS in Form einer wäßrigen Lösung und 0,09 g (0,4 mmol) Palladium(II)acetat, gelöst in 5 ml Toluol, zu. Die Mischung wird 15 Minuten gerührt. Die Reaktionsmischung wird auf 0°C gekühlt. Anschließend gibt man 6,24 g (0,06 mol) Natriumcarbonat, gelöst in 10 ml Wasser und 11,4 mg (0,6 mmol) Kupfer(I)iodid zu.

Die Reaktionsmischung wird 1 Stunde bei 0°C und 15 Stunden bei Raumtemperatur gerührt. Anschließend versetzt man mit 100 ml gesättigter wäßriger Ammoniumchloridlösung und 100 ml Ethylacetat. Die organische Phase wird abgetrennt und über Natriumsulfat getrocknet. Nach Einengen der organischen Phase wird das Rohprodukt aus 200 ml Methylcyclohexan bei -20°C umkristallisiert.
Ausbeute: 5,2 g (0,0313 mol; 78 % d.Th.); Schmelzpunkt: 74°C

### Beispiel 19:

9,5 g 1,4-Chloriodbenzol (0,04 mol) werden unter Inertgas in 50 ml Toluol gelöst. Man versetzt mit 25 ml Wasser und 25 ml Ethanol. Nacheinander gibt man 3,5 ml (0,06 mol) Propargylalkohol, 0,4 mmol BINAS in Form einer wäßrigen Lösung und 0,07 g (0,4 mmol) Palladium(II)chlorid, gelöst in 5 ml Toluol, zu. Die Mischung wird 15 Minuten gerührt. Die Reaktionsmischung wird auf 0°C gekühlt. Anschließend gibt man 6,24 g (0,06 mol) Natriumcarbonat, gelöst in 10 ml Wasser und 11,4 mg (0,6 mmol) Kupfer(I)iodid zu.

Die Reaktionsmischung wird 1 Stunde bei 0°C und 15 Stunden bei Raumtemperatur gerührt. Anschließend versetzt man mit 100 ml gesättigter wäßriger Ammoniumchloridlösung und 100 ml Ethylacetat. Die organische Phase wird abgetrennt und über Natriumsulfat getrocknet. Nach Einengen der organischen Phase wird das Rohprodukt aus 200 ml Methylcyclohexan bei -20°C umkristallisiert.
Ausbeute: 5,3 g (0,0319 mol; 80 % d.Th.); Schmelzpunkt: 74°C

### Beispiel 20:

9,5 g 1,4-Chloriodbenzol (0,04 mol) werden unter Inertgas in 50 ml Toluol gelöst. Man versetzt mit 25 ml Wasser und 25 ml Ethanol. Nacheinander gibt man 3,5 ml (0,06 mol) Propargylalkohol, 0,4 mmol BINAS in Form einer wäßrigen Lösung und 0,426 g (0,4 mmol) Palladium auf Aktivkohle (10 Gew.-%), suspendiert in 5 ml Toluol, zu. Die Mischung wird 15 Minuten gerührt. Die Reaktionsmischung wird auf 0°C gekühlt. Anschließend gibt man 5,1 g (0,06 mol) Piperidin und 11,4 mg (0,6 mmol) Kupfer(I)iodid zu.

Die Reaktionsmischung wird 1 Stunde bei 0°C und 15 Stunden bei Raumtemperatur gerührt. Anschließend versetzt man mit 100 ml gesättigter wäßriger Ammoniumchloridlösung und 100 ml Ethylacetat. Die organische Phase wird abgetrennt und über Natriumsulfat getrocknet. Nach Einengen der organischen Phase wird das Rohprodukt aus 200 ml Methylcyclohexan bei -20°C umkristallisiert.
Ausbeute: 6,1 g (0,0367 mol; 92 % d.Th.); Schmelzpunkt: 74°C

## Patentansprüche

1. Verfahren zur Herstellung von 1-Arylalkinen durch Kreuzkupplung von terminalen Alkinen mit aromatischen Halogenverbindungen oder - Perfluoralkylsulfonaten unter Palladiumkatalyse in Gegenwart mindestens eines wasserlöslichen Komplexliganden, dadurch gekennzeichnet, daß das Reaktionsmedium eine wäßrige und eine organische Phase bildet und das Palladium in Form einer in der organischen Phase löslichen Palladiumverbindung oder in fester Form als Palladiummetall zugesetzt wird.

2. Verfahren nach Anspruch 1, dadurch gekennzeichnet, daß eine in der organischen Phase lösliche Palladiumverbindung aus der Gruppe Palladiumketonate, Palladiumacetylacetonate, Nitrilpalladiumhalogenide, Olefinpalladiumhalogenide, Palladiumhalogenide, Allylpalladiumhalogenide und Palladiumbiscarboxylate eingesetzt wird.

3. Verfahren nach Anspruch 2, dadurch gekennzeichnet, daß eine in der organischen Phase lösliche Palladiumverbindung aus der Gruppe Bis(benzylidenaceton)palladium(0), Bis(benzylidenaceton)palladium(0)Chloroformkomplex, Palladiumbisacetylacetonat, Palladiumdichlorid, η³-Allylpalladiumchloriddimer, Natriumtetrachloropalladat, Dichloro(dimethylsulfoxid)palladium(II), Bis(benzonitril)palladiumdichlorid, Bis(acetonitril)palladiumdichlorid, Palladium-(II)-acetat, Palladium-(II)-propionat, Palladium-(II)-butanoat und (1c,5c-Cyclooctadien)palladiumdichlorid verwendet wird.

4. Verfahren nach Anspruch 1, dadurch gekennzeichnet, daß das Palladium als Palladium auf Aktivkohle, Palladium auf Aluminiumoxid, Palladium auf Bariumcarbonat, Palladium auf Bariumsulfat, Palladium auf Aluminiumsilikaten und/oder Palladium auf Calciumcarbonat eingesetzt wird.

5. Verfahren nach einem oder mehreren der vorhergehenden Ansprüche, dadurch gekennzeichnet, daß 0,001 bis 10 Mol-% Palladium, bezogen auf die aromatische Halogenverbindung oder das Perfluoralkylsulfonat, eingesetzt werden.

6. Verfahren nach einem oder mehreren der vorhergehenden Ansprüche, dadurch gekennzeichnet, daß als wasserlöslicher Komplexligand mindestens eine Verbindung aus der Gruppe Phosphane, Phosphite, Phosphonigsäureester, Phosphinigsäureester, Phosphole, Bipyridine, Phenanthroline, Porphyrine und Alizarine verwendet wird.

7. Verfahren nach Anspruch 6, dadurch gekennzeichnet, daß als wasserlösliche Komplexliganden wasserlösliche Phosphane der allgemeinen Formeln (I) bis (VII) eingesetzt werden, 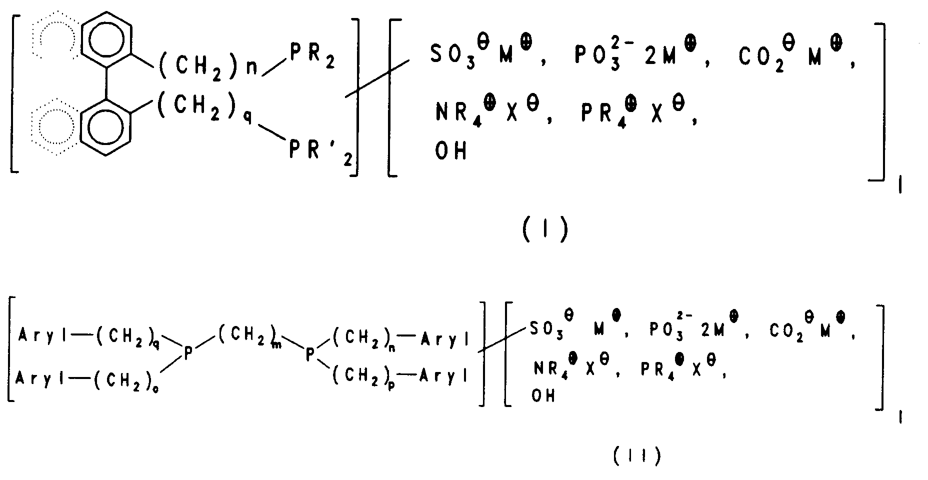 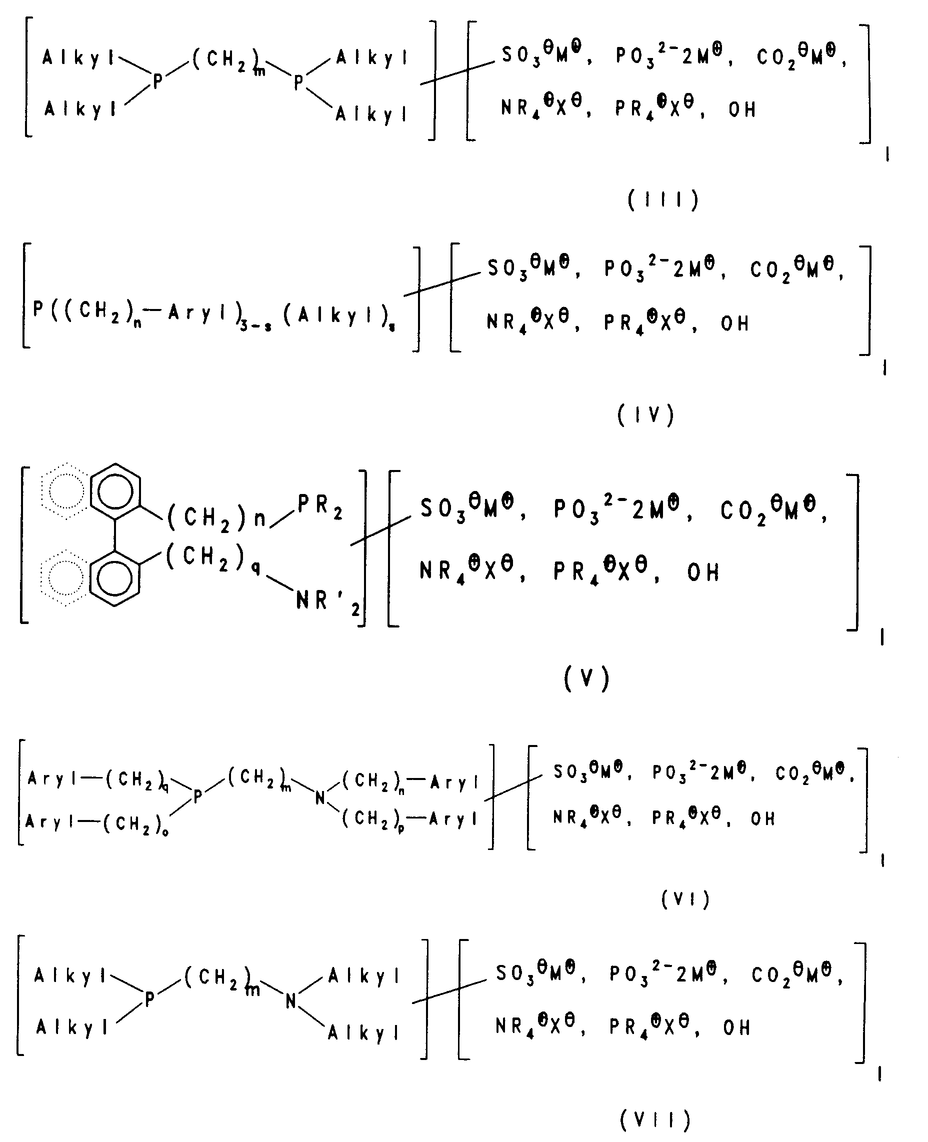 wobei die Symbole und Indizes folgende Bedeutungen haben:
Aryl: eine Phenyl oder Naphthyl, die auch einen oder mehrere Substituenten R tragen können;
Alkyl: eine geradkettige oder verzweigte Alkylgruppe mit 1 bis 8 Kohlenstoffatomen;
R,R': Alkyl, Aryl oder Aralkyl mit 1 bis 18 Kohlenstoffatomen;
M: Alkali-, Erdalkalimetall oder NR₄;
X: Halogen, BF₄, PF₆, OSO₂CF₃, 1/2[SO₄];
l,m: 1 bis 8;
n,o,p,q: 0, 1 bis 8;
s: 0, 1 bis 3.

8. Verfahren nach Anspruch 7, dadurch gekennzeichnet, daß als wasserlösliche Komplexliganden 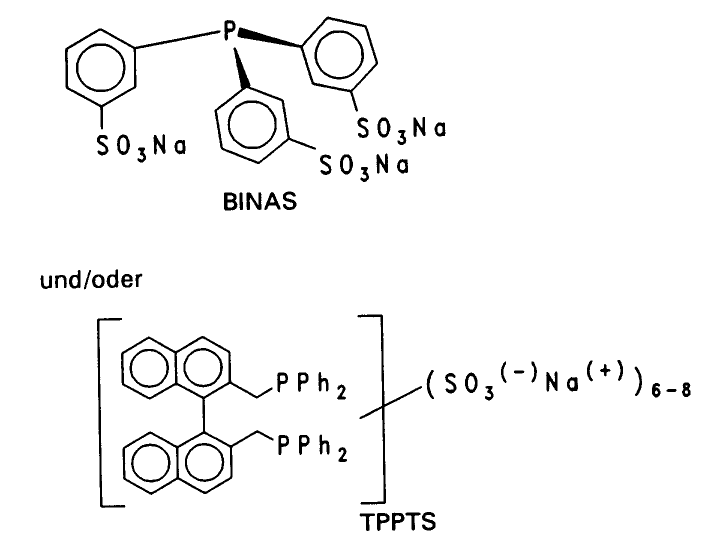 eingesetzt werden.

9. Verfahren nach einem oder mehreren der vorhergehenden Ansprüche, dadurch gekennzeichnet, daß der wasserlösliche Komplexligand in einem Anteil von 0,001 bis 20 Mol-%, bezogen auf die aromatische Halogenverbindung oder das Perfluoralkylsulfonat, eingesetzt wird.

10. Verfahren nach einem oder mehreren der vorhergehenden Ansprüche, dadurch gekennzeichnet, daß die organische Phase ein oder mehrere wasserunlösliche Lösungsmittel aus der Gruppe Kohlenwasserstoffe, Ether, höhere, mit Wasser nicht beliebig mischbare Alkohole, Ketone, Amide und Nitrile enthält.

11. Verfahren nach einem oder mehreren der vorhergehenden Ansprüche, dadurch gekennzeichnet, daß die wäßrige Phase der Reaktionsmischung ein wassermischbares organisches Colsolvens aus der Gruppe Nitrile, Amide und niedere Alkohole enthält.

12. Verfahren nach einem oder mehreren der vorhergehenden Ansprüche, dadurch gekennzeichnet, daß eine oder mehrere Kupfer(I)verbindungen als Cokatalysator eingesetzt werden.

13. Verfahren nach einem oder mehreren der vorhergehenden Ansprüche, dadurch gekennzeichnet, daß als Base mindestens eine Verbindung aus der Gruppe Alkali und Erdalkalimetallhydroxide, Alkali- und Erdalkalimetallcarbonate, Alkalimetallhydrogencarbonate, Alkali- und Erdalkalimetallacetate, Alkali- und Erdalkalimetallalkoholate und primäre, sekundäre und tertiäre Amine verwendet wird.

14. Verfahren nach einem oder mehreren der vorhergehenden Ansprüche, dadurch gekennzeichnet, daß es bei einer Temperatur von -10 bis 200°C durchgeführt wird.
